# EUROPEAN PATENT SPECIFICATION

(11) **EP 3 013 313 B1**
(45) Date of publication and mention of the grant of the patent: **26.12.2018**
(21) Application number: 14725296.9
(22) Date of filing: 31.03.2014
(51) Int. Cl.: A61K 8/38, A61K 8/37, A61Q 17/04, A61K 8/31, A61K 8/35, A61K 8/85

(54) **NON-IRRITATING, NON-WHITENING PHOTOPROTECTIVE COMPOSITIONS**
NICHT REIZENDE UND NICHT BLEICHENDE LICHTSCHUTZZUSAMMENSETZUNGEN
COMPOSITIONS PHOTOPROTECTRICES NON IRRITANTES, NON BLANCHISSANTES

(30) Priority: 01.04.2013 US 201361807089 P
(43) Date of publication of application: 04.05.2016
(73) Proprietor: Edgewell Personal Care Brands, LLC, Chesterfield, MO 63017 (US)
(72) Inventor: SPAULDING, Laura, Wayne, New Jersey 07470 (US); SANOGUERIA, James, Wesley Hills, New York 10901 (US); FRONTAURIA, Alissa, Lodi, New Jersey 07644 (US); LI, Geng, Rutherford, New Jersey 07070 (US)
(74) Representative: dompatent von Kreisler Selting Werner - Partnerschaft von Patent- und Rechtsanwälten mbB
(86) International application number: PCT/US2014/032325
(87) International publication number: WO 2015/152865

(56) References cited:
- WO-A1-2006/087066
- WO-A2-2007/024978
- WO-A2-2010/087962
- FR-A1- 2 904 220
- US-A1- 2007 092 475
- US-A1- 2010 323 042
- DATABASE GNPD [Online] MINTEL; December 2013 (2013-12), "pro series clear continuous spray sunscreen spf 30", XP002732795, Database accession no. 2271017
- DATABASE GNPD [Online] MINTEL; May 2012 (2012-05), "ultra light sunscreen lotion-spray spf 60", XP002732796, Database accession no. 1796306
- DATABASE GNPD [Online] MINTEL; April 2008 (2008-04), "ultra mist continuous spray sunblock spf 50", XP002732797, Database accession no. 898928
- DATABASE GNPD [Online] MINTEL; March 2012 (2012-03), "Lifting wrinkle treatment starter system", XP002732798, Database accession no. 1291551

## Description

This application claims priority under 35 U.S.C. §119(e) of co-pending U.S. Provisional Patent Application No. 61/807,089, filed on April 1, 2013, the disclosure of which is hereby incorporated by reference in its entirety.

### BACKGROUND OF THE INVENTION

### A. Field of Endeavor

The present invention relates to photoprotective compositions with additional mildness benefits, and more specifically, to clear, liquid, anhydrous based photoprotective compositions that are mild to both ocular and dermal regions of the body. The compositions of the invention are as defined in the set of claims.

### B. Background Information

Outdoor daytime activities such as swimming, fishing, exercising, landscaping, construction, children playing, etc., can expose the dermis of people participating in these activities to the damaging effects of ultraviolet (UV) exposure from both UVA and UVB radiation from the sun. Long term health effects are associated with early incidences of sunburn, and therefore, early use of photo-protective products should be encouraged for both young and older generations. Sunscreens are applied to the skin to protect the skin from the sun's ultraviolet rays that can lead to erythema (sunburn).

Sunlight or ultraviolet radiation in the UVB range has a wavelength of 290 nanometers to 320 nanometers and is known to be the primary cause of sunburn. Ultraviolet radiation at a wavelength of 320 nanometers to 400 nanometers, known as UVA radiation, can cause tanning of the skin. However, in the process of doing so, the UVA rays can damage or harm the skin.

Aside from the immediate malady of sunburn, excessive sunlight exposure can lead to other skin disorders. For instance, prolonged exposure to the sun may lead to actinic keratosis and carcinomas. Another long-term effect of sun exposure is premature aging of the skin. This condition is characterized by skin that is wrinkled, cracked and has lost its elasticity.

It is known that the combination of organic active ingredients providing broad spectrum photoprotection according to the 2011 United States Food and Drug Administration ("FDA") Sunscreen Monograph guidelines can cause ocular lacrimation and sting. Prior art commercially available products attempt to circumvent those effects by formulating with physical sunscreen active ingredients in a "whitening" thick/thin lotion format or as a "whitening" lotion spray.

Mintel (record ID 898928) discloses a spray sunblock spf 50 marketed as a tear-free sunblock that provides long lasting UVA/UVB protection. It is alcohol free but is an emulsion and comprises surfactants/emulsifiers, preservatives and silicone derivatives.

There is a long felt, unmet need to have clear photoprotective compositions that are mild to both ocular and dermal regions of the body. More specifically, there is a long, unfelt need to have photoprotective compositions that are mild enough to illicit no ocular lacrimation and/or no ocular sting, and/or no dermal irritation and/or no dermal sensitization.

Additionally, there is no clear and/or homogenous and/or anhydrous and/or alcohol free photoprotective composition that utilizes global regulatory body approved active agents that are both mild to both ocular and dermal regions of the body. Aerosol and bag on valve ("BOV") spray-type lotions are growing segment in the photoprotection marketplace due to dispensing convenience and more effective coverage of hard to reach areas when self-applying photoprotection product. The only Tear Free/Sting Free photoprotective aerosol or BOV product available to the consumer is an opaque, water-based lotion spray that needs to be shaken before use, and the liquid sprays on as an unsightly white film on wet or dry skin.

### SUMMARY

The present disclosure provides a photoprotective composition comprising at least one photoactive agent, at least one aliphatic hydrocarbon; and at least one ester. The compositions of the invention are as defined in the set of claims.

The present disclosure provides for a photoprotective composition that provides any or all of the following characteristics, including without limitation: is a single phase, is homogenous, is substantially non-whitening, is substantially non-irritating, is anhydrous, is water-resistant, is a skin-protectant, is substantially free of preservatives, is substantially free of surfactants, is substantially free of alcohol, is substantially free of emulsifiers, is substantially free of silicone, and any combinations thereof. The compositions are in the form of a flowing liquid that can be dispensed from a bottle with a suitable closure, or in a spray format such as an aerosol or pump spray.

In one embodiment of the present disclosure, the one or more photoactive agents are present in an amount about 0.1 wt. % to about 40 wt. %, based on the total weight of the composition. In another embodiment, the at least one aliphatic hydrocarbon is present in an amount of less than about 75 wt. %. In another embodiment, the at least one ester is present in an amount less than about 75%. In other embodiments, the at least one aliphatic hydrocarbon is between about 10 wt. % and about 75 wt. %. In another embodiment, the at least one ester is between about 20 wt. % and about 75 wt. %.

The photoprotective composition is a single-phase. In other words, the photoprotective composition does not have separate phases that could comprise without limitation, a water phase, an emulsion phase, and/or an oil phase.

In another aspect of the present disclosure, the photoprotective composition is homogenous. In other words, the product does not require, shaking, agitation, or other physical movement in order to dispense the photoprotective composition from a container such that the photoprotective composition is of a substantially uniform consistency.

The photoprotective composition is substantially non-whitening. In other words, the photoprotective composition is sufficiently translucent, transparent, clear, or any other adjective that describes a colorless or see-through substance. As hereinafter used, the term "non-whitening" is defined to include without limitation the aforementioned adjectives, such as, i.e., translucent, transparent, clear, colorless or see-through. The photoprotective composition is substantially non-whitening upon being dispensed from a container such that the photoprotective composition is substantially non-whitening when applied to a substrate, topically, to the dermis or skin, or any other surface. As hereinafter used, the terms "substrate", "topical", "dermis", "skin" and any other term referring to a surface on which a photoactive agent is applied, are all interchangeable. The photoprotective composition may also be substantially non-whitening after being topically applied and having sufficient time to dry. The photoprotective composition may also be substantially non-whitening when applied to substantially wet or substantially dry substrates, or combinations thereof.

The photoprotective composition is anhydrous.

In another aspect of the present disclosure, the photoprotective composition is water-resistant. The photoprotective composition may be suitable for sustaining water-resistance for at least forty (40) minutes. The photoprotective composition may be suitable for sustaining water-resistance for at least eighty (80) minutes. In any event, the photoprotective composition may be suitable to sustain a suitable level of photoprotection, such as but not limited to photoprotection achieving an SPF of 15, 30, 50 and/or 50+, as demonstrated by any or all of the FDA critical wavelength method, the UVAPF method, and the ISO24443 method, for a period of at least forty (40), and/or at least eighty (80) minutes.

The photoprotective composition is a skin-protectant.

In another aspect of the present disclosure, the photoprotective composition is able to achieve one or more of the aforementioned characteristics without resorting to other chemicals or substances. The present disclosure, the photoprotective composition does not contain preservatives. In other words, the photoprotective composition of the present disclosure is free of preservatives or preservative-free.

The photoprotective composition does not contain surfactants. In other words, the photoprotective composition of the present disclosure is free of surfactants or surfactant-free.

The photoprotective composition does not contain alcohol. In other words, the photoprotective composition of the present disclosure is free of alcohol or alcohol-free.

The photoprotective composition does not contain emulsifiers. In other words, the photoprotective composition of the present disclosure is free of emulsifiers or emulsifier-free.

The photoprotective composition does not contain silicone. In other words, the photoprotective composition of the present disclosure is free of silicone or silicone-free.

### BRIEF DESCRIPTION OF THE SEVERAL VIEWS OF THE DRAWINGS

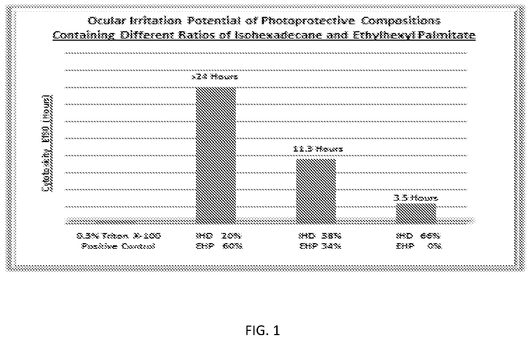

FIG. 1 is a chart showing ocular irritation potential of photoprotective compositions

### DETAILED DESCRIPTION OF THE INVENTION

The present disclosure will be discussed hereinafter in detail in terms of the preferred embodiments according to the present disclosure with reference to the accompanying drawings. In the following description, numerous, specific details are set forth in order to provide a thorough understanding of the present disclosure. It will be obvious, however, to those skilled in the art that the present disclosure may be practiced without these specific details. In other instances, well-known structures are not shown in detail in order to avoid unnecessary obscurity of the present disclosure.

The term "cosmetic composition" may include, but is not limited to, the following: sunscreens, dermatological compositions, make-up compositions, make-up removers, cleansers, lotions, gels, creams, sticks, balms, lip balms, powders, milks, conditioners, sprays, eye-liners, solutions, or serums. One of skill in the art understands that the photoprotective compositions of the present disclosure can facilitate cosmetic compositions.

The present disclosure is drawn to a photoprotective composition as defined in claim 1 that comprises at least one photoactive agent, at least one aliphatic hydrocarbon, and at least one ester.

The one or more photoactive agents comprises *p*-aminobenzoic acid (PABA), butyl methoxydibenzoylmethane, benzophenones, hydroxy-substituted benzophenones, methoxy-substituted benzophenones, benzophonone-1, benzophenone-2, benzophenone-3, benzophenone-4, benzophenone-6, benzophenone-8, benzophenone-12, methoxycinnamate, ethyl dihdroxypropyl-p-aminobenzoate, glyceryl-p-aminobenzoate, homosalate, methyl anthranilate, octocrylene, octyl dimethyl-p-aminobenzoate, octyl methoxycinnamate, octyl salicylate, 2 phenylbenzimidazole-5-sulphonic acid, triethanolamine salicylate, 3-(4-methylbenzylidene)-camphor, red petrolatum,3-(4-methylbenzyldine) bornan-2-one(methylbenzindinecamphor), benzotriazole, salicylates, phenylbenzimidazole-5-sulfonic acid, methylene bis-benzotriazolyl tetramethylbutyl phenol, avobenzone, butylmethoxydibenzoylmethane, octocrylene, octisalate, oxybenzone, bis-ethylhexyloxyphenol methoxyphenyl triazine, 4-isopropyl-dibenzoylmethane, metal oxides, zinc oxide, titanium dioxide, and any combinations thereof.

In one embodiment, the one or more photoactive agents are present in an amount about 0.1 wt. % to about 40 wt. %, based on the total weight of the photoprotective composition. In another embodiment, the one or more photoactive agents comprises: homosalate (trade name PARSOL® H MS) in amount less than or equal to 15 wt. %, octisalate (trade name PARSOL EHS) in an amount less than or equal to 5 wt. %, octocrylene (trade name PARSOL 340) in an amount less than or equal to 10 wt. %, avobenzone (PARSOL 1789) in an amount less than or equal to 5 wt. %, oxybenzone (trade name ESCALOL® 567) in an amount less than or equal to 0.5 wt. %, and bis-ethylhexyloxyphenol methoxyphenyl triazine (also known as bemotrizinol) (trade name TlNOSORB® S) in an amount less than or equal to 2.0 wt. %, based on the total weight of the photoprotective composition. In yet another embodiment, the photoprotective composition may be sufficient with only one or two or three photoactive agents. For instance, in one embodiment, oxybenzone is not needed in order to provide a photoprotective composition.

The at least one aliphatic hydrocarbon comprises isohexadecane (trade name PERMETHYL® 101A) or isododecane (trade name PERMETHYL 99A), and combinations thereof.

In one embodiment, the at least one aliphatic hydrocarbon is present in an amount less than or equal to about 75 wt. %, based on the total weight of the photoprotective composition. In another embodiment, the at least one aliphatic hydrocarbon is present in an amount of about 10 wt. % to about 75 wt. %, based on the total weight of the photoprotective composition. In yet another embodiment, the at least one aliphatic hydrocarbon is present in an amount between about 10 wt. % and about 50 wt. %, based on the total weight of the photoprotective composition. The aliphatic hydrocarbon comprises isohexadecane, isododecane, and combinations thereof.

The at least one ester comprises octyldodecyl citrate cross polymer (trade name COSMOSURF® CE-100), cetearyl ethylhexanoate (trade name SCHERCEMOL 1688), ethylhexyl palmitate (trade name CERAPHYL® 368), isononyl isononanoate (trade name DERMOL 99), octyldodecyl neopentanoate (trade name ELEFAC® 1-205), isodecyl neopentanoate (trade name BERNEL Ester 105), decyl oleate (trade name CERAPHYL 140), capric triglycerides and caprylic triglycerides such as those known by trade name DERMOL M5, isopropyl myristate (trade name DERMOL IPM), and combinations thereof.

In one embodiment, the at least one ester is present in an amount less than or equal to about 75 wt. %, based on the total weight of the photoprotective composition. In another embodiment, the at least one ester is present in an amount of about 20 wt. % to about 60 wt. %, based on the total weight of the photoprotective composition.

The determination of in-vitro static SPF was accomplished with Labsphere UV-2000S Transmittance Analyzer, Schonberg Sun PMMA HD2 plates, and the product application was scaled down to 1 mg/cm² for the signal to remain within the acceptable range of the instrument. Critical wavelength was accomplished in-house according to the guidelines set forth in the 2011 Sunscreen final rule, which is codified in the FDA regulations 21 C.F.R. 201.327. Instrumentation included the Labsphere UV 2000-S Transmittance Analyzer, Solar Simulator with 300W xenon arc lamp and a PMA 2100 radiometer with a UVB PMA 2101 Sensor. The substrate utilized was the Schonberg Sun PMMA HD2 plates. For the in-vitro FDA critical wavelength (CW) determination, the specified product application dose of 0.75 mg/cm² was used. The example prototype compositions shown below in Table 1 must minimally meet a critical wavelength of 370 nm to qualify for the broad spectrum protection claim, as shown in Table 2.

**Table 1**

| Composition (%) | A1 | A2 | A3 | A4 | A5 | A6 | A7 |
|---|---|---|---|---|---|---|---|
| Active Ingredients | | | | | | | |
| Homosalate | 9.00 | 9.00 | 9.00 | 9.00 | 9.00 | 9.00 | 10.00 |
| Octisalate | 5.00 | 5.00 | 5.00 | 5.00 | 5.00 | 5.00 | 5.00 |
| Octocrylene | 5.00 | 5.00 | 5.00 | 5.00 | 5.00 | 5.00 | 6.00 |
| Avobenzone | 3.00 | 3.00 | 3.00 | 3.00 | 3.00 | 3.00 | 3.00 |
| Isohexadecane | 73.48 | 65.48 | 73.48 | 20.00 | 40.00 | 38.00 | 20.00 |
| Ethylhexyl Palmitate | -- | -- | -- | 56.78 | 36.78 | 33.78 | 54.41 |
| EHMC | 1.00 | 1.00 | 1.00 | -- | -- | -- | -- |
| Polysilicone-15 | -- | 2.00 | -- | -- | -- | -- | -- |
| Other | q.s. 100 | q.s. 100 | q.s. 100 | q.s. 100 | q.s. 100 | q.s. 100 | q.s. 100 |

| | | | | | | | |
|---|---|---|---|---|---|---|---|
| EHMC = ethylhexyl methoxycrilene; compositions A1, A2 and A3 are not part of the invention as claimed. | | | | | | | |

**Table 2**

| Composition | *in vitro* SPF | *in vivo* SPF VWR | FDA CW |
|---|---|---|---|
| A1 | 111 ± 3 SD | 60.09 @ 95%CI 10 subjects | 370 |
| A2 | 122 ± 2 SD | -- | 370 |
| A3 | 110 ± 2 SD | -- | 370 |
| A4 | 106 ± 1 SD | -- | 372 |
| A5 | 105 ± 1 SD | -- | 371 |
| A6 | 105 ± 1 SD | -- | 371 |
| A7-1 | | 58.34 ± 0 SD 7 subjects | |
| | 108 ± 1 SD | | |
| A7-2 | | 56.93 @ 95%CI 10 subjects | 373 |

Dose for in vitro SPF Method: 1 mg/cm², Schonberg HD2 PMMA Plates
Dose for in vivo SPF VWR: 2 mg/cm², human subjects
Dose for FDA CW Method: 0.75 mg/cm², Schonberg HD2 PMMA Plates

As demonstrated by the embodiments of the photoprotective composition of the present disclosure, the photoprotective composition can provide a critical wavelength of greater than or equal to 370 nm.

The determination of in-vivo SPF very water resistance (VWR) was accomplished at Harrison Research, Laboratories, Union, NJ, according to the guidance provided in 21 CFR 201.327 specifying a product application dose of 2 mg/cm² on human subjects. Results indicated that SPF can be boosted with adjuvants such as ethylhexyl methoxycrylene and polysilicone-15. Both in vitro and in vivo SPF test results indicate that there is essentially no difference in UVR responses between the various ratios of isohexadecane and ethylhexyl palmitate. Additionally, according to the FDA critical wavelength test method, the tested embodiments of photoprotective compositions of the present disclosure meet the minimum 370 nm critical wavelength criteria, thus establishing the broad spectrum photoprotective nature of the present disclosure's photoprotective compositions.

Other embodiments of photoprotective compositions of the present disclosure as shown in Table 3 and their impact on SPF include those shown below in Table 4.

**Table 3**

| Composition (%) | B1 | B2 | B3 | B4 | B5 | B6 | B7 |
|---|---|---|---|---|---|---|---|
| Homosalate | 9.00 | 15.00 | 3.00 | 10.00 | 10.00 | 9.00 | 10.00 |
| Octisalate | 5.00 | 1.00 | 1.00 | 5.00 | 5.00 | 5.00 | 5.00 |
| Octocrylene | 5.00 | 10.00 | 10.00 | 5.00 | 5.00 | 9.00 | 5.00 |
| Avobenzone | 3.00 | 2.00 | 2.00 | 0.50 | 0.50 | 3.00 | 3.50 |
| Bemotrizinol | -- | -- | -- | 1.00 | 2.00 | -- | -- |
| Oxybenzone | -- | -- | -- | -- | -- | 0.5 | -- |
| Isohexadecane | 1.00 | 5.00 | 3.00 | 1.00 | 1.00 | 20.00 | 20.00 |
| Isododecane | 1.00 | 5.00 | 75.00 | -- | -- | -- | -- |
| Ethylhexyl Palmitate | 74.80 | 56.00 | -- | 72.00 | 64.50 | 52.10 | 48.30 |
| Butyloctyl Salicylate | 0.50 | 5.00 | 5.00 | 5.00 | 10.00 | -- | -- |
| Dimethicone | -- | 1.00 | 1.00 | -- | -- | -- | -- |
| Other | q.s. 100 | q.s. 100 | q.s. 100 | q.s. 100 | q.s. 100 | q.s. 100 | q.s. 100 |

**Table 4**

| Composition | *in vitro* SPF | λ nm @0.5 Abs | ISO 24443 in vitro UVA |
|---|---|---|---|
| B1 | 107 ± 5 SD | 387.0 | -- |
| B2 | 115 ± 2 SD | 386.0 | -- |
| B3 | 114 ± 4 SD | 386.0 | -- |
| B4 | 112 ± 1 SD | 384.0 | Does Not Meet 1/3 UVA Ratio |
| B5 | 124 ± 2 SD | 384.5 | Does Not Meet 1/3 UVA Ratio |
| B6 | 122 ± 2 SD | 387.5 | -- |
| B7 | 108 ± 2 SD | 388.5 | Meets 1/3 UVA Ratio |

Compositions B2 and B3 are not part of the invention as claimed. comparison of photoprotective compositions A1 and B1 further support the ratios of isohexadecane, isododecane, and ethylhexyl palmitate are interchangeable with essentially no effect on the in vitro SPF value. The bemotrizinol prototype compositions did not meet the 1/3 UVA ratio according to the ISO 24443 method due to the SPF boosting provided by the addition of butyloctyl salicylate. Unexpectedly, the B7 embodiment of the photoprotective composition achieved the optional 1/3 UVA ratio and the SPF VWR 60. Not only did the composition score a UVAPF value of 25.7 (0.42 UVA/SPF), but also exhibited an ISO 24443 method post-irradiation critical wavelength of 377 nm. In one embodiment, the photoprotective composition meets the 1/3 UVA ratio requirement of ISO 24443.

The present disclosure's photoprotective composition is provided such that the composition is a single phase. The photoprotective composition is mixed in one stage (i.e., a single phase) such that multiple phases or stages where certain water, emulsion or oil phases are required in order to obtain a suitable photoprotective composition are unnecessary. As such, the number of steps that are required in manufacturing the photoprotective composition of the present disclosure are reduced.

In another embodiment, the photoprotective composition is homogenous such that the product does not require shaking, agitation, or other physical movement in order to dispense the photoprotective composition from a container such that the photoprotective composition is of a substantially uniform consistency. Homogeneity can be facilitated by embodiments of the present disclosure that are single phase, but homogeneity can be achieved with liquids that are not a single phase.

The photoprotective composition is substantially non-whitening. The photoprotective composition is substantially non-whitening upon being dispensed from a container such that the photoprotective composition is substantially non-whitening when applied to a substrate. In other words, the photoprotective composition is substantially non-whitening upon removal from a canister, upon topical application, upon a significant length of time passing after topical application, or any combinations thereof. The photoprotective composition may also be substantially non-whitening after being topically applied and having sufficient time to dry. The photoprotective composition may also be substantially non-whitening when applied to substantially wet or substantially dry substrates, or combinations thereof. Said another way, the photoprotective composition is substantially non-whitening when topically applied to a substantially wet substrate, to a substantially dry substrate, or combinations thereof.

A substantially non-whitening composition can be quantified by visual perception studies including the ability to identify/ascertain something lying below/underneath a composition. For instance and for purposes of the present disclosure, a substantially non-whitening liquid is defined by the ability to read font size of 10 point or less printed on a substrate onto which a liquid is applied. Preferably and for the purposes of the present disclosure, a substantially non-whitening liquid visual perception study requires being able to read a font size of 4 to 6 through 7,62cm (three (3) inches) of liquid. The following scale is exemplary:
This is 10 point font
This is 8 point font
This is 6 point font
This is 4 point font

In one embodiment, the photoprotective composition is substantially non-whitening such that 10 point Arial font can be read through 7,62cm (three (3) inches) of the photoprotective composition liquid. In another embodiment, the photoprotective composition is substantially non-whitening such that 8 point Arial font can be read through 7,62cm (three (3) inches) of the photoprotective composition liquid. More preferably, in another embodiment, the photoprotective composition is substantially non-whitening such that 6 point Arial font can be read through 7,62cm (three (3) inches) of the photoprotective composition liquid. Most preferably, an embodiment of the photoprotective composition is substantially non-whitening such that 4 point Arial font can be read through 7,62cm (three (3) inches) of the photoprotective composition liquid.

Another means of ascertaining of whether or not a liquid is substantially non-whitening is by how the liquid visually appears when applied to a substantially wet substrate, a substantially dry substrate, or combinations thereof. In other words, the liquid should not produce an opaque film upon (a) initial application of the liquid to the substrate, regardless of whether the substrate is wet, dry or combinations thereof, (b) upon being lathered, oscillated, or rubbed onto, over and/or into the substrate, (c) after a sufficient time has passed between either or both of (a) and (b) such that the substrate has had a chance to dry or settle into a finally-applied state on the substrate. In one embodiment, the photoprotective composition does not produce an opaque film upon initial application to a substrate, wherein the substrate is substantially wet, substantially dry, or combinations thereof. In another embodiment, the photoprotective composition does not produce an opaque film upon lathering, oscillation, rubbing onto, over or into the substrate, wherein the substrate is substantially wet, substantially dry, or combinations thereof. In another embodiment, the photoprotective composition does not produce an opaque film after the photoprotective composition has had sufficient time to dry after (a) initial application to a substantially wet substrate, a substantially dry substrate, or combinations thereof, (b) lathering, oscillation or rubbing onto, over and into a substantially wet substrate, a substantially dry substrate, or combinations thereof, or both (a) and (b). In a preferred embodiment, the photoprotective composition never leaves an opaque film upon application to a substantially wet substrate, a substantially dry substrate, or combinations thereof.

With regard to the photoprotective compositions of the present disclosure, it was observed that high levels of isohexadecane, especially those of about 70 wt. %, limited the solubility and/or compatibility of other ingredients which cause undesirable characteristics within the entire photoprotective composition such as, i.e., hazing, oily segregation, and/or precipitation from the composition, and combinations thereof. Limited solubility was observed with polyester materials and their diluents of propylene glycol dibenzoate and neopentyl glycol heptanoate. Dimethicone with a viscosity of about 350 cst at a 1 wt. % concentration, for example, causes hazing. A suitable amount of diluent for avobenzone became critical as crystals appeared in stability samples after storage for three (3) months at 40°C. As such, finding a photoprotective composition that is substantially non-irritating and also does not cause other deleterious effects (i.e., affecting homogeneity, affecting mixing of chemicals thereby instigating separation, clouding or whitening the liquid, etc...) is difficult.

The photoprotective composition is substantially non-irritating. The photoprotective composition is substantially non-irritating in that it is mild, or in other words, it elicits substantially no dermal irritation, such as but not limited to, i.e., sensitization, erythema, stinging, burning, itching, eczema, flaking, drying, other skin conditions and combinations thereof. In other words, the photoprotective composition is substantially non-irritating such that upon topical application, minimal dermal irritation is elicited. Dermal irritation or sensitization can be quantitatively and qualitatively ascertained through human clinical trials known as Human Repeat Insult Patch Testing (HRIPT). The HRIPT trial(s) comprise a repeat insult patch test with a minimum of two-hundred (200) human subjects. Each human subject is qualified and thereafter participates in the study.

Such HRIPT tests were conducted regarding several embodiments of the photoprotective composition of the present disclosure. The testing facility used for purposes of the present disclosure was Clinical Research Laboratories, Inc., Piscataway, NJ (CRL). Prior to applying a patch, the test area was wiped with 70% isopropyl alcohol and allowed to dry. The test material was applied to the upper back (between the scapulae) and was allowed to remain in direct skin contact for a period of twenty-four (24) hours. Patches were applied to the same site on Monday, Wednesday and Friday, for a total of nine (9) applications during the induction period. The sites were graded by a CRL technician for dermal irritation twenty-four (24) hours after removal of the patches by the subjects on Tuesday and Thursday and forty-eight (48) hours after removal of the patches on Saturday, unless the patching schedule was altered as described above. If a 2+ reaction or greater occurred, the test material was applied to an adjacent virgin site. If a 2+ reaction or greater occurred on the new site, the subject was not patched again during the induction phase but was challenged on the appropriate day of the study. At the discretion of the Study Director, patch sites with scores less than a 2+ may have been changed. Following approximately a two (2) week rest period, the challenge patches were applied to previously untreated test sites on the back. After twenty-four (24) hours, the patches were removed by a CRL technician and the test sites were evaluated for dermal reactions. The test sites were re-evaluated at forty-eight (48) and seventy-two (72) hours. Subjects exhibiting reactions during the challenge phase of the study may have been asked to return for a ninety-six (96) hour reading. During the induction period and the challenge phase, test sites were graded by CRL according to the following scoring system:

### Dermal Scoring Scale:

0 = No visible skin reaction
+ = Barely* Perceptible Erythema
1+ = Mild erythema
2+ = Well defined erythema
3+ = Sever Erythema and edema
4+ = Erythema and edema with vesiculation

Also noted were other skin conditions such as dryness, itching, papules, hyperpigmentation, etc...

*Due to subjectivity and extreme randomness, the rating was discounted and regarded as background noise.

Results from the HRIPT indicated that in the photoprotective compositions, the lower levels (≤ 40 wt. %) of isohexadecane are preferred to achieve a substantially non-irritating composition. In one embodiment, a substantially non-irritating composition is also substantially non-whitening.

**Table 5**

| **Human Dermal Clinical Trial Results** | | | | |
|---|---|---|---|---|
| | **IHD** | **EHP** | **Irritation** | **Sensitization** |
| A3 | 73.48 % | 0.00 % | Several Incidences | Possibly One |
| A4 | 20.00 % | 56.78 % | No Incidences | No Incidences |
| A5 | 40.00 % | 36.78 % | No Incidences | No Incidences |

| | | | | |
|---|---|---|---|---|
| IHD = isohexadecane EHP = ethylhexyl palmitate | | | | |

In another embodiment, the present disclosure's photoprotective composition is substantially non-irritating in that it is mild, or in other words, it elicits substantially no ocular irritation, such as but not limited to, i.e., non-lacrimating (non-tearing, tear-free); does not induce stinging, burning, or itching; does not induce ocular, bulbar conjunctival irritation, palpebral conjunctival irritation, or any combinations thereof. In other words, the photoprotective composition is substantially non-irritating such that upon topical application, minimal ocular irritation is elicited. Ocular irritation can be qualitatively and quantitatively ascertained by EpiOcular™ Human Cell construct testing. Such testing was done at the Institute for in Vitro Sciences. The MTT (3-(4,5-dimethylthiazol-2-yl)-2,5-diphenyltetrazolium bromide) conversion assay, which means the NAD(P)H-dependent microsomal enzyme reduction of MTT (and to a lesser extent, the succinate dehydrogenase reduction of MTT) to a blue formazan precipitate, was used to assess cellular metabolism after exposure to the test article for various exposure times. The duration of exposure resulting in a 50% decrease in MTT conversion in test article-treated EpiOcular™ human cell constructs, relative to control cultures, was determined (ET50). The positive control for the studies was 0.3% Triton-X-100 (i.e. an irritating composition), and the negative control was sterile deionized water.

As exemplified in the embodiments tested in FIG. 1, the concentration of isohexadecane was unexpectedly found to influence how little dermal irritation was elicited. It was further unexpectedly discovered that a correlation between isohexadecane and ethylhexyl palmitate (isohexadecane: ethylhexyl palmitate) also influences eliciting substantially no dermal irritation. In other words the ratio of isohexadecane (IHD) to ethylhexyl palmitate (EHP) affects the amount, or lack thereof, of dermal irritation that is elicited.

It should be noted that the toxicologist's conclusion for the EpiOcular™ test results indicated the prototype samples were non-irritating to the human cell construct versus the Triton X-100 positive control.

For purposes of the present disclosure's photoprotective composition, Human Clinical Trials were conducted at Harrison Research Laboratories, Inc., Union, NJ (HRL). These trials were controlled, randomized and double-blinded. Twenty (20) human subjects were qualified and thereafter participated in the trial. Each subject's eyes were examined by the Ophthalmological Investigator immediately before treatment. At all stages of the procedure, the subject and the Ophthalmological Investigator were unaware of which eye was treated with which Test Material and of the previous assessment scores. Sterile disposable eye swab was used for the right eye of the subject for instillation of the appropriate Test Material or Control as per the randomization schedule. A new sterile disposable eye swab was used for the left eye. Each instillation was performed by a trained HRL technician. Two white tissues (one for each eye) were provided to blot any tears which fell from the eye. Within thirty (30) seconds, or as closely possible following instillation, the eyes were examined by the Ophthalmological Investigator and graded according to the Scoring Scale, shown below. The eyes were examined and scored again at five (5) minutes, followed by the washing of the eyes to flush them out. The eyes were again examined and scored at fifteen (15) minutes and one (1) hour post-instillation. If residual reactions had remained, the Ophthalmological Investigator would have flushed the eyes with an irrigation solution. Any subject who exhibited a 1-level or higher score at one (1) hour would not have been dismissed until he or she returned to a zero (0) level score. During the study, the subjects were not permitted to apply any product directly to the eyes, eyelids or eyelashes, and were seated in a secure area.

To evaluate embodiments of photoprotective compositions for causing ocular irritation, visual and physical sensory indicators such as ocular lacrimation and sting can be used as evaluation criteria. The below scoring system demonstrates the stringent passing criteria imposed at each of the timed intervals shown in FIG. 1 throughout the study, where the first criteria is based on lacrimation (i.e., tearing of the eyes) and the second criteria is stinging.

### Lacrimation- Ophthalmologist Scoring Scale

0 = Within normal limits
1 = Excessive wetness (no distinct tears)
2 = A few formed tears (contained in orbit)
3 = Intense tearing (leave orbit)

In a twenty (20) subject human study, the maximum (i.e. worst) score is sixty (60) points at each time interval (i.e. after thirty (30) seconds, after five (5) minutes, after fifteen (15) minutes, and after sixty (60) minutes). In other words, twenty (20) subjects multiplied by a worst score for all subjects of three (3) points, yields a total score of sixty (60) points. A passing score for no lacrimation is less than or equal to four (4) points to account for lack of distinction between real and subjectivity randomness. Note the rigid standard requiring less than or equal to four (4) point score while the scale provides for sixty (60) points. In other words, achieving a lacrimation score of less than or equal to four (4) is incredible in view of prior art formulations.

### Stinging (Pain, etc...) Self Perceived Scoring Scale

0 = Within normal limits
1 = Mild, very slight
2 = moderate
3 = severe

In a twenty (20) subject human study, the maximum (i.e. worst) score is sixty (60) points at each time interval (i.e. after thirty (30) seconds, after five (5) minutes, after fifteen (15) minutes, and after sixty (60) minutes). A passing score for no stinging is less than or equal to four (4) points to account for lack of distinguishing between real and subjectivity randomness. Note the rigid standard requiring less than or equal to four (4) point score while the scale provides for sixty (60) points. In other words, achieving a stinging score of less than or equal to four (4) is unexpected and incredible in view of prior art formulations. Furthermore, achieving a lacrimation score of less than or equal to four (4) and a stinging score of less than our equal to four (4) is further unexpected and remarkable.

Results from the two (2) human ocular clinical trials are summarized below. The results for the twenty (20) subjects per each trial indicate that both compositions passed, indicating the composition is substantially non-irritating to the eyes as the photoprotective composition embodiments tested showed scores indicating substantially no ocular lacrimation was elicited and substantially no ocular stinging was elicited. In other words, the photoprotective compositions of the present disclosure are substantially non-irritating in that there is substantially no ocular lacrimation and/or substantially no ocular stinging.

**Table 6**

| **Human Ocular Clinical Trial Results** | | | | |
|---|---|---|---|---|
| | **IHD** | **EHP** | **Lacrimation** | **Sting** |
| A1 | 73.48 % | 0.00 % | Pass 0/20 | Pass 4/20 |
| A7 | 20.00 % | 54.41 % | Pass 0/20 | Pass 1/20 |

Due to the subjectivity factor, it is unclear if the lower concentration of isohexadecane produced the milder sting score. One skilled in the art understands that embodiments with higher levels of isohexadecane, as demonstrated in tested embodiment A1, can provide the unexpected result of a passing ocular lacrimation and/or sting study. The present disclosure has unexpectedly found that certain hydrocarbons in certain concentrations contribute to a substantially non-irritating photoprotective composition. It has been further unexpectedly found that certain hydrocarbons not only provide substantially no dermal irritation or substantially no ocular irritation, and further still, no ocular lacrimation and/or no ocular sting, but are capable of providing substantially non-irritating compositions to both dermal and ocular regions. Preferably, the present disclosure has found light to medium weight hydrocarbons with a viscosity of about 2 CST to about 100 CST at 40°C (as per the ASTM D-445 standard) contribute to substantially non-irritating photoprotective compositions. Preferably, these hydrocarbons are highly emollient, light weight and slightly volatile such as but not limited to, i.e. isohexadecane, isododecane, and other aliphatic hydrocarbons such as but not limited to, i.e. mineral oil, waxes, C₁₃ to C₂₂ alkanes, and C₁₂ to C₁₆ isoparaffins.

Further surprisingly and unexpectedly, the tested embodiments of photoprotective compositions A7 plus 0.2% fragrance (the modified composition is test photoprotective composition A8) performed well in the "wet skin" in vivo SPFVWR test. In this study, ten (10) human subjects were pre-immersed for five (5) minutes to hydrate the skin, and then the photoprotective composition was applied immediately to wet skin so that air drying could not occur between immersion and application steps. The human subjects were then treated according to the FDA SPFVWR assay. The results as demonstrated in Table 7 below indicate that the tested embodiments of the photoprotective compositions of the present disclosure performed very well on both wet and dry skin for a product with an SPF rating of 50+.

**Table 7**

| **Composition** | **Protocol** | ***in vivo* SPF** |
|---|---|---|
| A7 | FDA SPF VWR | 56.93 @ 95%CI 10 subjects |
| A8 | Pre-Immersion Prior to FDA SPF VWR | 56.14 @ 95%CI 10 subjects |

As shown above, some embodiments of the photoprotective composition are water-resistant. In one embodiment, the photoprotective composition meets the Sun Protection Factor Very Water Resistance threshold. In another embodiment, the photoprotective composition may be suitable for sustaining water-resistance for at least forty (40) minutes. In a further embodiment, the photoprotective composition may be suitable for sustaining water-resistance for at least eighty (80) minutes. In other embodiments the photoprotective composition may be suitable to sustain a suitable level of photoprotection, as demonstrated by any or all of the FDA critical wavelength method, the UVAPF method, and/or the ISO24443 method, for a period of forty (40), and/or eighty (80) minutes. In further embodiments, the photoprotective composition is capable of sustaining an FDA SPF rating of 15, 30, 50, or 50+, for a period of forty (40), and/or eighty minutes (80) under the FDA VWR test method.

A photoprotective composition is substantially non-whitening and is also substantially non-irritating. In some of these embodiments, the photoprotective composition is substantially non-whitening on a substantially dry substrate. In other of these embodiments, the photoprotective composition may be substantially non-whitening on a substantially wet substrate. In other embodiments, the photoprotective composition may be substantially non-whitening on a substantially wet substrate and a substantially dry substrate. In some of these embodiments, the photoprotective composition is substantially non-whitening upon dispensing from a container and upon application to a substrate. In further embodiments, the photoprotective composition is also substantially non-whitening after a sufficient amount of time has elapsed in order to permit the photoprotective composition to dry onto, over and/or into the substrate. In yet further embodiments, the photoprotective composition are substantially non-whitening upon being rubbed, lathered, and oscillated onto, over and/or into the substrate. Preferably, the photoprotective composition of these embodiments is completely non-whitening. In any of these embodiments and as noted above, the photoprotective composition may also be substantially non-irritating such that is causes substantially no dermal irritation. In any of these embodiments, the photoprotective composition may also be substantially non-irritating in that it causes substantially no ocular irritation. In further embodiments, the photoprotective composition may also be substantially non-irritating in that it causes substantially no ocular lacrimation, substantially no ocular sting, or both. In yet further embodiments, the photoprotective composition is preferably non-irritating. In yet further embodiments, the photoprotective composition preferably causes no ocular lacrimation, causes no ocular sting, causes no dermal irritation, or any combinations thereof. In further embodiments, the photoprotective composition may also be homogenous, a single phase, anhydrous, water-resistant, or any combinations thereof. One skilled in the art appreciates how surprising and unexpected achieving a photoprotective composition with any single characteristic as described throughout is. One skilled in the art truly appreciates the challenge in balancing any number or plurality of characteristics as described throughout, while still maintaining a sufficient level of photoprotection. One skilled in the art has further appreciation for the challenges of accomplishing a photoprotective composition that is, for instance, i.e., non-irritating and/or non-whitening, may further be a single phase and/or is homogenous and/or anhydrous, and achieving this without (i.e., such that the photoprotective composition is substantially free of) alcohol, preservatives, further active ingredients (i.e., oxybenzone), surfactants, other chemicals, or any combinations thereof.

The photoprotective composition is free of preservatives, of surfactants, of alcohol, of emulsifiers and of silicone but can include other ingredients i.e., solvents, emollients, skin protectants, additional film forming and/or water proofing agents, SPF boosters, vitamins and/or antioxidants, thickening agents, moisturizers, humectants, bio-actives, pH adjuster/chelating agents, fragrances, effect pigments, color additives, lubricants, elastomers, and any combinations thereof.

several embodiments of the photoprotective compositions of the present disclosure were internally tested for microbial content: the containers were tested prior to filling the containers with the photoprotective compositions and after filling. The embodiments tested include A1, A2, and A7; all of these tested embodiments were found to contain no colonies of total plate count and on enrichment plates, and thus passed the internal test. In other words, although at least one preservative may be prove beneficial, the present disclosure provides for embodiments that do not require preservatives.

In yet another embodiment, a film former/waterproofing agent is selected from the group consisting of mineral oil, lotus japonicas symbiosome extract, C₁₄ to C₂₂ alkanes, C₁₃ to C₁₆ isoparaffins, C₁₂ to C₁₄ isoparaffins, C₁₃ to C₁₅ alkanes, polyester-10, propylene glycol dibenzoate, polyester-7, neopentyl glycol diheptanoate, polyester-17, and combinations thereof.

One skilled in the art that the application is not so limited by the trade names provided throughout the present disclosure and that components, chemicals, materials, additives, constituents and any other ingredient can be of similar composition to those listed and can be sourced from other products by different trade names or brands than those noted throughout the present disclosure.

In one aspect of the present disclosure, the photoprotective composition may be easier to manufacture. The key components of the photoprotective composition of the present disclosure inherently possess oil solubility (i.e., are lipophilic). The constituents of the photoprotective compositions of the present disclosure may be amphipathic. As such, embodiments of the present disclosure can be manufactured at ambient conditions and thus don't require elevated temperatures for manufacture. In some embodiments, heat may optionally be applied in order to reduce processing time, but increased temperature or heat is not necessary otherwise. In other embodiments of the present disclosure, manufacturing can be shortened from a time standpoint if solid ingredients (i.e., avobenzone, bemotrizinol, waxes, and other ingredients that are substantially solid at room temperature) are mixed with liquids and then heated to melt temperature. Adding liquids may also be used to cool the batch quickly, and as such, one skilled in the art will appreciate that the photoprotective compositions of the present disclosure can provide fewer processing steps, fewer processing constraints (i.e., ambient temperature may be sufficient), and combinations thereof.

In various embodiments, the photoprotective composition can be filled into any number of bottles, tottles, tubes having a variety of closing means, pumps, sprays (i.e., aerosols, bag on valve), amongst others, and combinations thereof. Aerosol embodiments can be filled with various propellants and combinations of propellants, depending on requirements for that product. Propellants that may be used include but are not limited to, i.e., isobutene, isobutene/propane, dimethyl ether, tetraflouroethane, and 1,1-difluoroethane. Ratios of propellant to the photoprotective composition concentrate can be adjusted to account for cost, aesthetics, and product performance objectives.

Alcohol is not necessary. Alcohols often elicit dermal and/or ocular irritation (i.e., stinging and/or burning) and as such, are not required for purposes of the present disclosure. In view of the absence of alcohol, embodiments of the present disclosure may be more suitable for sprays including both aerosol and bag on valve systems. Embodiments without alcohol are potentially less-flammable than formulations containing alcohol.

In one embodiment, at least one propellant may be used such as, but not limited to, i.e., isobutene, propane, dimethyl ether, tetraflouroethane, 1,1-difluoroethane, and combinations thereof.

In one embodiment, the photoprotective composition concentrate and propellant are added to an aerosol container in a ratio of about 70:30 to about 60:40. The tetraflouroethane and difluoroethane propellants were tested and are non-flammable in view of the flame extension test.

In another embodiment, the photoprotective composition concentrate and propellant are added to an aerosol container in a ratio of about 70:30. The tetraflouroethane and isobutene propellants were evaluated to determine what blends of tetraflouroethane and isobutene propellants would provide a non-flammable product at a reasonable cost. In other words, it was found that a lowest ratio of tetraflouroethane to isobutene propellant that achieved no flame extension was a ratio of about 3:1. In some embodiments having a photoprotective composition comprising a concentrate and at least one propellant, the ratio of the photoprotective composition and the propellant can be between about 2.5:1 and about 1.25:1.

In order to evaluate overall performance of photoprotective compositions of the present disclosure, sensory and consumer testing can be done and benchmarked against existing products. In one such in-house study with between seven (7) and ten (10) panelists, several embodiments of the photoprotective compositions of the present disclosure were tested, including two compositions containing high levels of isohexadecane (73.4 wt. % and 74.98 wt. %) and two different silicone blends (cyclopentasiloxane/cyclohexasiloxane, polymethylsilsesquioxane/ethylmethicone), which were compared to a control of the currently sold BANANA BOAT® SPORT Lotion Spray that claims to be tear-free and sting-free. The aforementioned embodiments of the present disclosure's photoprotective compositions were substantially non-whitening upon application and upon being rubbed-in; the currently sold BANANA BOAT SPORT Lotion Spray currently sold was found to whiten upon being rubbed-in. Feedback from the ten (10) panelists noted the embodiments of the present disclosure's photoprotective compositions that were tested were significantly shinier, greasier and/or oily on the skin. All three compositions tested contained isobutene propellant.

In a second in-house study with between seven (7) and ten (10) panelists, different embodiments of the present disclosure's photoprotective composition were tested: an embodiment comprised 37.68 wt. % isohexadecane, 34.00 wt. % ethylhexyl palmitate and cyclopentasiloxane/cyclohexasiloxane. The ratio of concentrate to propellant was 60:40; the propellants evaluated were isobutene and dimethyl ether. The control product was once again the currently sold BANANA BOAT SPORT Lotion Spray. Upon applying to dry skin, the embodiment comprising dimethyl ether was surprisingly found to be significantly shinier and more oily and/or greasier than the embodiment comprising dimethyl ether. When applied to wet skin, the currently sold BANANA BOAT SPORT Lotion Spray was more difficult to rub-in and/or spread, and furthermore, whitened the skin. Surprisingly, the embodiments of the present disclosure were found to be easy to rub-in and/or spread, and were substantially non-whitening. As such, in one embodiment, it may be preferable to have a concentrate to propellant ratio of 60:40, wherein the propellant is at least partially comprised of dimethyl ether. While this embodiment may provide, upon application, a product that is less shiny, less greasy and/or less oily, one of skill in the art understands that all exemplary embodiments of the present disclosure's photoprotective compositions demonstrate substantially non-whitening products.

In another study, sixty (60) consumers were provided embodiments of the photoprotective compositions of the present disclosure for home-use. One embodiment comprised 20 wt. % isohexadecane, 54.1 wt. % ethylhexyl palmitate, and no siloxanes. The concentrate to propellant ratio of this embodiment tested was 60:40, and the propellant evaluated was dimethyl ether. The control product was the currently sold BANANA BOAT SPF 50 Kids bag-on-valve product. So as to prevent fragrance halo effects, fragrance was added to the test embodiment. Results from the study showed test embodiment of the photoprotective composition of the present disclosure was preferred based on the following characteristics/factors:

| | |
|---|---|
| Overall liking: | Preferred at 80% confidence limit (CL) |
| Skin feel, liking rubbing-in: | Preferred at 90% CL |
| Did not feel sticky or tacky: | Preferred at 90% CL |
| Did not turn white when sprayed on wet skin: | Preferred at 90% CL |
| Skin feel softness: | Preferred at 80% CL |
| Skin feel smoothness: | Preferred at 90% CL |

As such, one skilled in the art understands that the above-mentioned tested embodiments demonstrate the photoprotective composition of the present disclosure provide a significantly improved consumer experience, as identified in the above-mentioned sensory characteristics/factors. One skilled in the art further understands that the present disclosure has unexpectedly achieved a photoprotective composition that provides for one or more of these sensory characteristics/factors, one or more of the physical attributes and/or characteristics that provides for the sensory results shown above, and that a plurality of embodiments, as demonstrated above and throughout the present disclosure can achieve the desired performance criteria acknowledged throughout the present disclosure.

## Claims

1. An anhydrous, single-phase and skin-protecting photoprotective composition comprising:
at least one photoactive agent selected from the group consisting of p-aminobenzoic acid, butyl methoxydibenzoylmethane, benzophenones, hydroxy-substituted benzophenones, methoxy-substituted benzophenones, benzophonone-1, benzophenone-2, benzophenone-3 (oxybenzone), benzophenone-4, benzophenone-6, benzophenone-8, benzophenone-12, methoxycinnamate, ethyl dihydroxypropyl-p-aminobenzoate, glyceryl-p-aminobenzoate, homosalate, methyl anthranilate, octocrylene, octyl dimethyl-p-aminobenzoate, octyl methoxycinnamate, octyl salicylate (octisalate), 2-phenylbenzimidazole-5-sulphonic acid, triethanolamine salicylate, 3-(4-methylbenzylidene)-camphor, red petrolatum, 3-(4-methylbenzyldine)bornan-2-one(methylbenzindinecamphor), benzotriazole, phenylbenzimidazole-5-sulfonic acid, methylene bis-benzotriazolyl tetramethylbutyl phenol, butylmethoxydibenzoylmethane (avobenzone), octocrylene, bis-ethylhexyloxyphenol methoxyphenyl triazine, 4-isopropyl-dibenzoylmethane, metal oxides, zinc oxide, titanium dioxide, and any combinations thereof;
at least one aliphatic hydrocarbon selected from the group consisting of isohexadecane, isododecane, and combinations thereof; and
at least one ester selected from the group consisting of octyldodecyl citrate cross polymer, cetearyl ethylhexanoate, ethylhexyl palmitate, isononyl isononanoate, octyldodecyl neopentanoate, isodecyl neopentanoate, decyl oleate, capric triglycerides, caprylic triglycerides, isopropyl myristate, and combinations thereof;
wherein said photoprotective composition is free of preservatives, free of surfactants, free of alcohol, free of emulsifiers, and free of silicone; and
wherein said photoprotective composition is non-whitening, is non-irritating, or both.

2. The photoprotective composition of claim 1, wherein said at least one photoactive agents are present in an amount 0.1 wt. % to 40 wt. %, based on a total weight of the photoprotective composition, preferably wherein said at least one photoactive agents comprises: homosalate in amount less than or equal to 15 wt. %, octisalate in an amount less than or equal to 5 wt. %, octocrylene in an amount less than or equal to 10 wt. %, avobenzone in an amount less than or equal to 5 wt. %, oxybenzone in an amount less than or equal to 0.5 wt. %, and bis-ethylhexyloxyphenol methoxyphenyl triazine in an amount less than or equal to 2.0 wt. %, based on a total weight of the photoprotective composition.

3. The photoprotective composition of claim 1 or 2, wherein said at least one aliphatic hydrocarbon is present in an amount less than or equal to 75 wt. %, based on the total weight of the photoprotective composition, preferably wherein said at least one aliphatic hydrocarbon is present in an amount of 10 wt. % to 75 wt. %, based on the total weight of the photoprotective composition.

4. The photoprotective composition of any of the preceding claims, wherein said at least one ester is present in an amount less than or equal to 75 wt. %, based on the total weight of the photoprotective composition, preferably wherein said at least one ester is present in an amount of 20 wt. % to 60 wt. %, based on the total weight of the photoprotective composition.

5. The photoprotective composition of claim 1 comprising:
at least one photoactive agents in an amount of 0.1 wt. % to 40 wt. % and selected from the group consisting of homosalate, octisalate, octocrylene, avobenzone, oxybenzone, and combinations thereof;
at least one aliphatic hydrocarbon in an amount of less than 75 wt. %; and
at least one ester in an amount of less than 75 wt. %;
wherein said photoprotective composition is tear-free.

6. The photoprotective composition of claim 5, wherein the at least one ester comprises ethylhexyl palmitate.

## Patentansprüche

1. Wasserfreie einphasige hautschützende Lichtschutzzusammensetzung, umfassend:
wenigstens ein photoaktives Mittel, das aus der Gruppe ausgewählt ist, die aus p-Aminobenzoesäure, Butylmethoxydibenzoylmethan, Benzophenonen, hydroxysubstituierten Benzophenonen, methoxysubstituierten Benzophenonen, Benzophenon-1, Benzophenon-2, Benzophenon-3 (Oxybenzon), Benzophenon-4, Benzophenon-6, Benzophenon-8, Benzophenon-12, Methoxycinnamat, Ethyldihydroxypropyl-p-aminobenzoat, Glyceryl-p-aminobenzoat, Homosalat, Methylanthranilat, Octocrylen, Octyldimethyl-p-aminobenzoat, Octylmethoxycinnamat, Octylsalicylat (Octisalat), 2-Phenylbenzimidazol-5-sulfonsäure, Triethanolaminsalicylat, 3-(4-Methylbenzyliden)campher, Rohvaseline, 3-(4-Methylbenzyliden)bornan-2-on (Methylbenzylidencampher), Benzotriazol, Phenylbenzimidazol-5-sulfonsäure, Methylenbis(benzotriazolyltetramethylbutylphenol), Butylmethoxydibenzoylmethan (Avobenzon), Octocrylen, Bis-ethylhexyloxyphenolmethoxyphenyltriazin, 4-Isopropyldibenzoylmethan, Metalloxiden, Zinkoxid, Titandioxid und irgendwelchen Kombinationen davon besteht;
wenigstens einen aliphatischen Kohlenwasserstoff, der aus der Gruppe ausgewählt ist, die aus Isohexadecan, Isododecan und Kombinationen davon besteht; und
wenigstens einen Ester, der aus der Gruppe ausgewählt ist, die aus Octyldodecylcitrat-Kreuzpolymer, Cetearylethylhexanoat, Ethylhexylpalmitat, Isononylisononanoat, Octyldodecylneopentanoat, Isodecylneopentanoat, Decyloleat, Caprinsäuretriglyceriden, Caprylsäuretriglyceriden, Isopropylmyristat und Kombinationen davon besteht;
wobei die Lichtschutzzusammensetzung frei von Konservierungsstoffen, frei von Tensiden, frei von Alkohol, frei von Emulgatoren und frei von Silikon ist; und
wobei die Lichtschutzzusammensetzung nichtweißend, nichtreizend oder beides ist.

2. Lichtschutzzusammensetzung gemäß Anspruch 1, wobei das wenigstens eine photoaktive Mittel in einer Menge von 0,1 Gew.-% bis 40 Gew.-% vorhanden ist, bezogen auf das Gesamtgewicht der Lichtschutzzusammensetzung, wobei das wenigstens eine photoaktive Mittel vorzugsweise umfasst: Homosalat in einer Menge von weniger oder gleich 15 Gew.-%, Octisalat in einer Menge von weniger oder gleich 5 Gew.-%, Octocrylen in einer Menge von weniger oder gleich 10 Gew.-%, Avobenzon in einer Menge von weniger oder gleich 5 Gew.-%, Oxybenzon in einer Menge von weniger oder gleich 0,5 Gew.-% und Bisethylhexyloxyphenolmethoxyphenyltriazin in einer Menge von weniger oder gleich 2,0 Gew.-%, bezogen auf das Gesamtgewicht der Lichtschutzzusammensetzung.

3. Lichtschutzzusammensetzung gemäß Anspruch 1 oder 2, wobei der wenigstens eine aliphatische Kohlenwasserstoff in einer Menge von weniger oder gleich 75 Gew.-% vorhanden ist, bezogen auf das Gesamtgewicht der Lichtschutzzusammensetzung, wobei vorzugsweise der wenigstens eine aliphatische Kohlenwasserstoff in einer Menge von 10 Gew.-% bis 75 Gew.-% vorhanden ist, bezogen auf das Gesamtgewicht der Lichtschutzzusammensetzung.

4. Lichtschutzzusammensetzung gemäß einem der vorstehenden Ansprüche, wobei der wenigstens eine Ester in einer Menge von weniger oder gleich 75 Gew.-% vorhanden ist, bezogen auf das Gesamtgewicht der Lichtschutzzusammensetzung, wobei vorzugsweise der wenigstens eine Ester in einer Menge von 20 Gew.-% bis 60 Gew.-% vorhanden ist, bezogen auf das Gesamtgewicht der Lichtschutzzusammensetzung.

5. Lichtschutzzusammensetzung gemäß Anspruch 1, umfassend:
wenigstens ein photoaktives Mittel in einer Menge von 0,1 Gew.-% bis 40 Gew.-% und ausgewählt aus der Gruppe, die aus Homosalat, Octisalat, Octocrylen, Avobenzon, Oxybenzon und Kombinationen davon besteht;
wenigstens einen aliphatischen Kohlenwasserstoff in einer Menge von weniger als 75 Gew.-%; und
wenigstens einen Ester in einer Menge von weniger als 75 Gew.-%;
wobei die Lichtschutzzusammensetzung nicht augenreizend ist.

6. Lichtschutzzusammensetzung gemäß Anspruch 5, wobei der wenigstens eine Ester Ethylhexylpalmitat umfasst.

## Revendications

1. Composition photo-protectrice anhydre, monophasée et de protection de la peau comprenant :
au moins un agent photo-actif choisi dans le groupe constitué par l'acide p-aminobenzoïque, le méthoxydibenzoylméthane de butyle, les benzophénones, les benzophénones à substitution hydroxy, les benzophénones à substitution méthoxy, la benzophonone-1, la benzophénone-2, la benzophénone-3, la benzophénone-4, la benzophénone-6, la benzophénone-8, la benzophénone-12, le méthoxycinnamate, le dihydroxypropyl-p-aminobenzoate d'éthyle, le glycéryl-p-aminobenzoate, l'homosalate, l'anthranilate de méthyle, l'octocrylène, le diméthyl-p-aminobenzoate d'octyle, le méthoxycinnamate d'octyle, le salicylate d'octyle (octisalate), l'acide 2-phénylbenzimidazole-5-sulfonique, le salicylate de triéthanolamine, le 3-(4-méthylbenzylidène)-camphre, la vaseline rouge, le 3-(4-méthylbenzyldine)bornan-2-one(méthylbenzindincamphre), le benzotriazole, l'acide phénylbenzimidazole-5-sulfonique, le méthylène bis-benzotriazolyl tétraméthylbutyl phénol, le butylméthoxydibenzoylméthane (avobenzone), l'octocrylène, la bis-éthylhexyloxyphénol-méthoxy-phényl-triazine, le 4-isopropyldibenzoylméthane, des oxydes métalliques, l'oxyde de zinc, le dioxyde de titane et toute combinaison de ceux-ci ;
au moins un hydrocarbure aliphatique choisi dans le groupe constitué par l'isohexadécane, l'isododécane et des combinaisons de ceux-ci ; et
au moins un ester choisi dans le groupe constitué par le polymère croisé de citrate d'octyldodécyle, l'éthylhexanoate de cétéaryle, le palmitate d'éthylhexyle, l'isononanoate d'isononyle, le néopentanoate d'octyldodécyle, le néopentanoate d'isodécyle, l'oléate de décyle, les triglycérides capriques, les triglycérides capryliques, le myristate d'isopropyle, et des combinaisons de ceux-ci ;
dans lequel ladite composition photo-protectrice est exempte d'agents de conservation, exempte d'agents tensioactifs, exempte d'alcool, exempte d'agents émulsifiants et est exempte de silicone ; et
où ladite composition photo-protectrice est non blanchissante, non irritante, ou les deux.

2. Composition photo-protectrice selon la revendication 1, dans laquelle ledit au moins un agent photo-actif est présent en une quantité de 0,1 % en poids à 40 % en poids, par rapport au poids total de la composition photo-protectrice, où de préférence ledit au moins un agent photo-actif comprend : de l'homosalate en une quantité inférieure ou égale à 15 % en poids, de l'octisalate en une quantité inférieure ou égale à 5 % en poids, de l'octocrylène en une quantité inférieure ou égale à 10 % en poids, de l'avobenzone en une quantité inférieure ou égale à 5 % en poids, de l'oxybenzone en une quantité inférieure ou égale à 0,5 % en poids et de la bis-éthylhexyloxyphénol-méthoxyphényl-triazine en une quantité inférieure ou égale à 2,0 % en poids, par rapport au poids total de la composition photo-protectrice.

3. Composition photo-protectrice selon la revendication 1 ou 2, dans laquelle ledit au moins un hydrocarbure aliphatique est présent en une quantité inférieure ou égale à 75 % en poids, par rapport au poids total de la composition photo-protectrice, de préférence dans laquelle ledit au moins un hydrocarbure aliphatique est présent en une quantité de 10 % en poids à 75 % en poids, par rapport au poids total de la composition photo-protectrice.

4. Composition photo-protectrice selon l'une quelconque des revendications précédentes, dans laquelle ledit au moins un ester est présent en une quantité inférieure ou égale à 75 % en poids, par rapport au poids total de la composition photo-protectrice, de préférence dans laquelle ledit au moins un ester est présent en une quantité de 20 % en poids à 60 % en poids, par rapport au poids total de la composition photo-protectrice.

5. Composition photo-protectrice selon la revendication 1, comprenant :
au moins un agent photo-actif en une quantité de 0,1 % en poids à 40 % en poids et choisi dans le groupe consistant en l'homosalate, l'octisalate, l'octocrylène, l'avobenzone, l'oxybenzone et des combinaisons de ceux-ci ;
au moins un hydrocarbure aliphatique en une quantité inférieure à 75 % en poids, et
au moins un ester en une quantité inférieure à 75 % en poids ;
dans laquelle ladite composition photo-protectrice est non irritante pour les yeux.

6. Composition photo-protectrice selon la revendication 5, dans laquelle le au moins un ester comprend le palmitate d'éthylhexyle.
